# EUROPEAN PATENT APPLICATION

(11) **EP 2 664 614 A1**
(43) Date of publication of application: **20.11.2013**
(21) Application number: 11855523.4
(22) Date of filing: 30.12.2011
(51) Int. Cl.: C07C 319/24, B01J 31/16, B01J 23/89, A61K 31/095, A61K 31/28, A61K 31/30, A61P 31/12, A61P 7/04, A61K 38/06

(54) **PALLADIUM-COPPER CATALYSTS FOR THE HOMOGENEOUS SELECTIVE OXIDATION OF THIOL GROUPS**

(30) Priority: 11.01.2011 RU 2011101479
(71) Applicant: "Ivy Pharm" Limited Liability Company, St.Petersburg 190121 (RU)
(72) Inventor: BALAZOVSKY, Mark Borisovich, St.Petersburg 199106 (RU); ANTONOV, Viktor Georgievich, Vsevolozhsk Leningradskaya Oblast 188643 (RU); BELYAEV, Alexandr Nikolaevich, St.Petersburg 192212 (RU); EREMIN, Alexei Vladimirovich, St.Petersburg 198260 (RU)
(74) Representative: Jennings, Tara Romaine
(86) International application number: PCT/RU2011/001055
(87) International publication number: WO 2012/096595

(57) **Abstract**

A palladium-copper catalyst of homogeneous selective oxidation of thiols is proposed, combining a functional binuclear thiolate-bridged coordination compound of palladium (II) and a modifying thiolate complex of copper (I), having the general formula

[Pd₂^{II}(µ-SR)₂(NH₃)₄] · {Cu^{I}ₖ(SR)ₘ} (I),

where
SR is the residue of a thiolate ligand, chosen from the group including a residue of glutathione and acetylcysteine,
k = 2 to 14,
m ≥ 3k.

Also proposed are a catalytic combination, a pharmacological combination, a pharmaceutical composition, and a method of therapeutic action on a patient's organism based on the indicated catalyst.

## Description

### Field of technology of the invention

The present invention pertains to bio-inorganic chemistry, medical chemistry, and medicine, specifically, to the field of preparation of medicinal products, and it can be used in bio-inorganic chemistry, pharmacology, medicine and veterinary practice.

### Prior art

Boosting the therapeutic effectiveness of pharmacological molecules by optimizing their pharmacokinetics and/or pharmacodynamics and/or reducing the toxicity by chemical modification of the molecule of a pharmaceutical agent and/or using it together with other chemical compound(s) is one of the areas for creating a new generation of pharmaceutical products that present their activity in more physiologically optimal doses.

Thus, a number of combination agents are known, including Amoxiclav, which contains amoxicillin and clavulanic acid in its makeup, and Tienam, containing imipenem in combination with cilastatin, a specific inhibitor of the kidney enzyme dihydropeptidase. Clavulanic acid prevents the breakdown of amoxicillin by bacterial enzymes, while cilastatin inhibits the metabolism of imipenem in the kidneys, which substantially increases the concentration of the unaltered antibiotic in the kidneys and urinary tracts.

However, there are many other pharmaceutical agents that are potentially useful for the treatment of illness, but do not provide the desired effect due to the developing of one or another form of resistance to them.

At present, the substance N-glutamyl-L-cysteinyl-glycine disulfide (GSSG) or oxidized glutathione is known, which in itself possesses various pharmacological activity. In particular, oxidized glutathione has been found to be able to initiate processes carrying out various kinds of chemical modification: phosphorylation, glutathionylation, oxidation and others, which precede the formation of a particular structural conformation with a high affinity for the ligand and ability to perform a physiological function. Oxidized glutathione has been shown to be able to intensify the production of a broad spectrum of cytokines, which control a complex of protective reactions of the body, including antiviral, antibacterial, antitumoral, and antifibrotic action A number of pharmacological solutions has been proposed for the creation of composites including a complex compound of oxidized glutathione and cisplatin in combination with pharmacologically active molecules for treatment of various diseases, including diabetes, ischemic heart disease, viral hepatitis, malignant tumors, suppurating infections, and a number of others. In particular, a pharmacological solution has been proposed for treatment of drug-resistant forms of viral hepatitis B and C to intensify the antiviral activity of inosine, used in the form of an organic salt with oxidized glutathione (RU 2153350, RU2153351).

However, the makeup of the oxidized glutathione product disclosed in RU 2153350 includes cisplatin, which is a complex compound of platinum (Pt), whose use is coupled with the danger of a toxic and mutagenic action. It is indeed the platinum which manifests a catalytic effect when it is used in a minimal quantity.

The danger of toxic action of platinum in the makeup of the oxidized glutathione preparation when given repeatedly has been proven in an experiment on cells, where a daily introduction of the complex platinum compound over the course of five days resulted in suppression of the proliferative activity of a culture and its death. The use of oxidized glutathione also imposes certain limitations on the possible pharmacological solutions, since it enables the creation of medicinal forms of primarily parenteral administration

Therefore, the need exists to develop new products having the ability to boost the therapeutic effectiveness of pharmacological molecules by optimizing their pharmacokinetics and/or pharmacodynamics that are suitable for creation of medicinal forms of enteral, parenteral and other possible methods of administration.

### Essence of the invention

This problem is solved by proposing a palladium-copper catalyst of homogeneous selective oxidation of thiols that combines a functional binuclear thiolate-bridged coordination compound of palladium (II) and a modifying thiolate complex of copper (I), having the general formula

[Pd₂^{II}(µ-SR)₂(NH₃)₄] · {Cu^{I}ₖ(SR)ₘ} (I),

where
SR is the residue of a thiolate ligand, chosen from the group including a residue of glutathione and acetylcysteine,
k = 2 to 14,
m ≥ 3k.

Preferably the oxidation is a homogeneous selective oxidation of thiols with forming of disulfide bonds between the thiol residues, while the thiol whose oxidation is subjected to a catalytic function is N-acetyl-cysteine or N-glutamyl-L-cysteinyl-glycine.

Preferably the catalyst is obtained by the reaction of mononuclear aminate complexes of palladium (II) and corresponding thiols with complexes forming from salts of copper (II) and corresponding thiols.

Advisedly, the molar ratio of Pd:Cu in the catalyst of the invention lies in the range of 1:0.1 to 1:2, more preferably in the range of 1:0.2 to 1:1.

The catalyst of the invention can be used in therapy.

Also proposed is a catalytic combination formed by a thiol chosen from among acetylcysteine, glutathione, their solvates and salts, and by the catalyst of the invention

Preferably the catalyst is present in the combination in a quantity of 1·10⁻² to 1·10⁻⁷ g per mole of thiol. The proposed combination can essentially consist only of acetyl cysteine disulfide and/or glutathione, their solvates and salts, and the catalyst of the invention.

The combination of the invention can be used in therapy.

Also proposed is a pharmacological combination, including the indicated catalytic combination and a pharmacologically active compound able to enter into an addition reaction with the components of the combination.

It is advisable to use this combination to boost the therapeutic activity of the pharmacologically active compound

The indicated pharmacologically active compound can be a medicinal or biologically active molecule chosen from the purine or pyrimidine bases or their derivatives.

This combination can be used in the treatment of infectious and noninfectious diseases. The pharmacologically active compound can be, for example, ribavirin.

Also proposed is a pharmaceutical composition, including the described catalyst or combination and a pharmaceutically acceptable excipient. Such a pharmaceutical composition boosts the therapeutic activity of the pharmacologically active compounds.

Also proposed is a method of therapeutic action on a patient's organism to boost the therapeutic activity of a pharmacologically active compound, wherein the patient requiring this is given an effective quantity of the catalyst, the combination or the composition according to the invention.

### Description of graphic materials

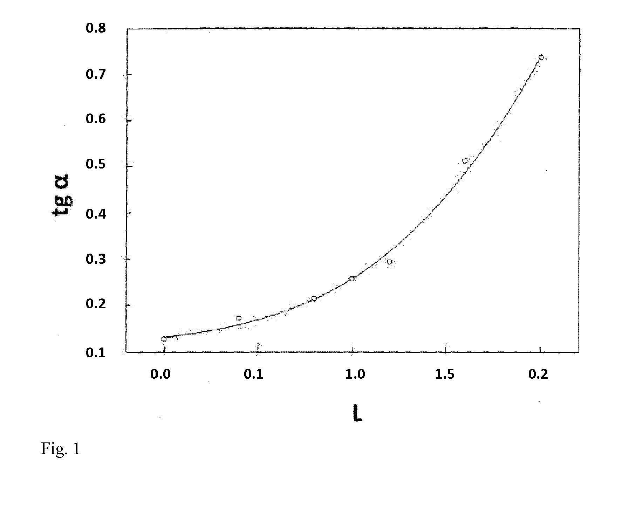

Figure 1 shows the accumulation of N-glutamyl-L-cysteinyl-glycine disulfide as a function of the ratio of the number of moles of Pd:Cu in the system: "GSH - H₂O₂-[Pd^{II}₂(µ-SG)₂(NH₃)₄]·{Cu^{I}ₙ(SR)ₘ}" (tg(α) is the slope for the reaction of glutathione oxidation by hydrogen peroxide in the presence of the Pd-Cu catalyst, L=n(Cu)/n(Pd), 25±0.1° C, C_{GSH} 2 mg/ml, pH 6.0, C_{pd} 3.4e-6 mole/liter).

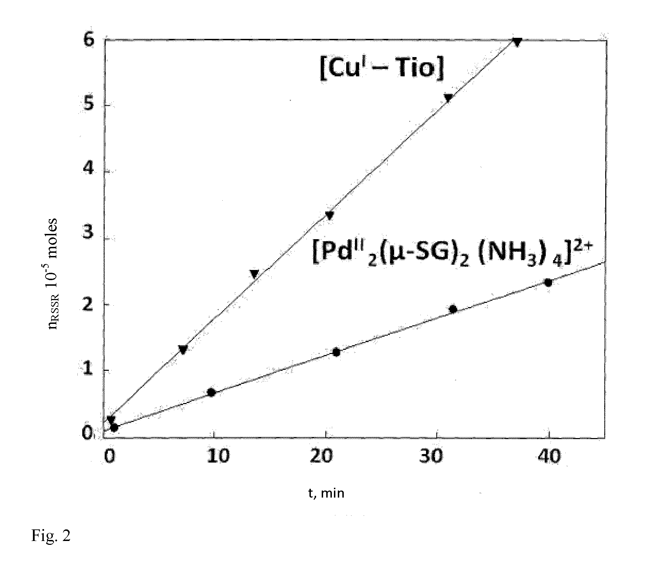

Figure 2 demonstrates the relative catalytic effectiveness of {Cu^{I}ₖ(SR)ₘ} complexes in the reaction of oxidation of N-glutamyl-L-cysteinyl-glycine by hydrogen peroxide as compared to the binuclear palladium complex [Pd₂(µ-SG)₂(NH₃)₄]²⁺ (25:1:0.1° C, C_{GSH} = 2 mg/ml, pH = 5.3, C_{M} = 6.5e-6 mole/liter).

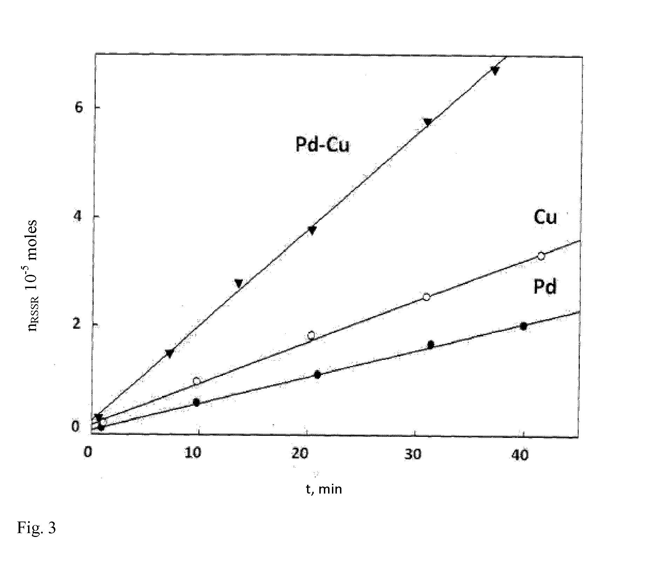

Figure 3 presents the curves of oxidation ofN-glutamyl-L-cysteinyl-glycine by palladium and copper complexes and by binary Pd-Cu catalyst (25±0.1° C, C_{GSH} 2 mg/ml, pH 6.0, C_{M} 6.3e-6 mole/liter).

### Detailed specification of the invention

The efforts of the inventors to use the preparation N-glutamyl-L-cysteinyl-glycine disulfide obtained without the use of cisplatin or with the use of another metal resulted in lower therapeutic benefits.

The inventors discovered that many of the effects of the pharmacological activity of N-glutamyl-L-cysteinyl-glycine disulfide, obtained by the method in RU 2153350, are connected to the ability of the preparation to bring about a catalytic oxidation of sulfhydryl groups to disulfides in the composition of molecules of a peptide nature.

The inventors identified a need to conduct a controllable catalysis, which has been achieved with the help of the proposed catalyst of the invention

Thiolate complexes of copper (I), being added to binuclear thiolate-bridged coordination compounds of palladium (II), are able to significantly change the rate, but not the extent of oxidation of a thiol. As an example, Fig. 1 shows curves of the accumulation ofN-glutamyl-L-cysteinyl-glycine disulfide (GSSG) as a function of the Pd:Cu ratio in the system "GSH - H₂O₂ - [Pd^{II}₂(µ-SG)₂(NH₃)₄]·{Cu^{I}ₖ(SR)ₘ}".

As can be seen from the results presented in Fig. 1, the catalytic effectiveness of the palladium catalyst modified by copper (I) ions increases as the Pd:Cu ratio varies from 1:0 to 1:2.

It is well known that Cu²⁺ ions are themselves able to effectively catalyse the oxidation of thioamino acids to form -S-S- bonds in them. Therefore, to ascertain the region of change of the Pd:Cu ratio in the proposed Pd-Cu binary catalytic system, the inventors considered separately the catalytic effectiveness of binuclear thiolate-bridged complexes of palladium (II), thiolate complexes of copper (I), and their interworking.

### Oxidation of thiols by binuclear palladium complexes

In the catalytic oxidation of thiols RSH, one of the most important functions of palladium is the forming of coordination complexes - products of the addition of thiolate ions RS⁻, to the palladium ion with their subsequent oxidation and disruption of the coordination polyhedron.

The spatial proximity of the thiolate ions entering as ligands into the coordination sphere of a metal can be easily achieved in binuclear palladium (II) complexes in which the thiolate ions RS⁻ take up a bidentate bridge coordination, resulting in the formation of a Pd₂^{II}(µ-SR)₂ skeleton.

For the oxidation of thiolate ions, we considered a catalytic cycle with the participation of a binuclear hydroxo-bridged ammonium complex of palladium (II) - [Pd₂(µ-OH)₂(NH₃)₄]²⁺, for which the following reactions were examined as being the dominant ones:

[Pd₂(µ-OH)₂(NH₃)₄]²⁺+2RSH. [Pd₂(µ-SR)₂(NH₃)₄]²⁺+2H₂O (5)

[Pd₂(µ-SR)₂(NH₃)₄]²⁺+H₂O₂ . {[Pd₂(µ-SR)₂(NH₃)₄(OH)₂]²⁺}^{#} (6)

{[Pd₂(µ-SR)₂(NH₃)₄(OH)₂]²⁺}^{#}. [Pd₂(µ-OH)₂(NH₃)₄]²⁺+RSSR (7)

Equations (5) and (7) constitute stages in which the catalyst [Pd₂(µ-OH)₂(NH₃)₄]²⁺ is consumed and regenerated once again. Reaction (6) is the main stage by which the formation of an unstable intermediate palladium complex {[Pd₂(µ-SR)₂(NH₃)₄(OH)₂]²⁺} is possible.

Quantum chemistry calculations revealed that, in the oxidation of the binuclear metallic skeleton pd₂^{II}(µ-SR)² by hydrogen peroxide, the most energy-efficient in the intermediate bimetallic compound is an addition of both OH groups to a single palladium atom, making it possible to consider the intermediate as a binuclear mixed-valency complex with the skeleton Pd^{II}Pd^{IV}(µ-SR)₂ This skeleton, when reduced, in turn oxidizes the thiolate bridge groups.

The quantum chemistry calculations of the coordination compounds were carried out by the method of DFT B3LYP in a 6-31G** base by the program Jaguar 7.5. For the Pd atoms, we used the effective pseudopotential of the HW skeleton with corresponding valency base. Analysis of the frequencies of the normal oscillations revealed that all structures of compounds obtained by optimization of the geometry correspond in the gas phase to minima on the potential energy surface. The energies of solvation of the compounds were calculated in the polarizable continuum model. To reduce the number of basic functions, the molecules of glutathione, acetylcysteine, or thioglycolic acid RSH were modeled by the most elementary thiol, CH₃SH.

The reason for the instability of the intermediate coordination compound Pd^{II}Pd^{IV} is related to the presence of an oxidizer (the ion Pd^{IV}) and reducing agents (the ligands µ-SR) in its internal sphere, which leads to an intrasphere redox process. This process includes a synchronous transfer of two electrons from the pair of thiol coordination bridges to the ion Pd^{IV}, resulting in the breaking of the Pd-SR bridge bonds and the unification of two thiol radicals RS· into a disulfide R₂S₂. Afterwards, in the coordination sphere of the reduced palladium dimer, there occurs an intramolecular regrouping of the ligands OH⁻, previously coordinated to Pd^{IV}, into a bridge position. The result is the formation of the compound [Pd(NH₃)₂(µ-OH)]₂²⁺, which is the start of the catalytic cycle under consideration (scheme 1).

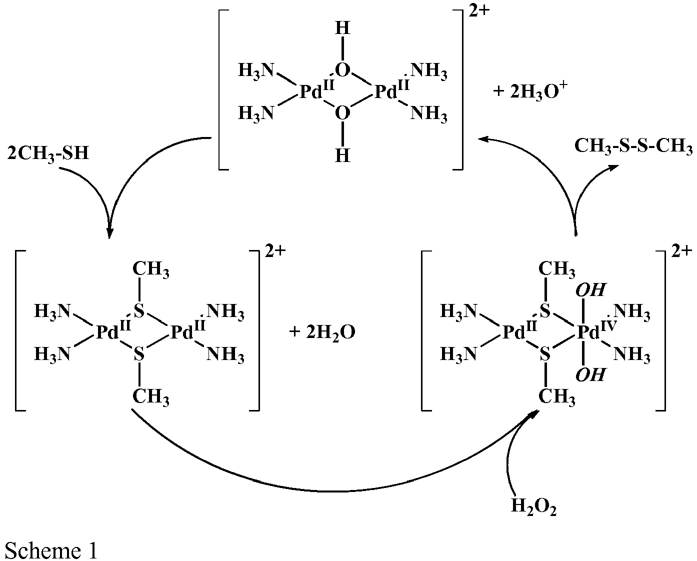

It should be noted that in the absence of hydrogen peroxide (O₂ or any other oxidizing agents), aqueous solutions of binuclear thiolate-bridged palladium (II) complexes do not catalyse the oxidation of thioamino acid.

An evaluation of the effectiveness of the catalytic action of the compound [Pd₂(µ-SG)₂(NH₃)₄]²⁺ on the process of oxidation of glutathione (GSH) by hydrogen peroxide, carried out by means of the method of high-efficiency liquid chromatography (HELC), revealed a greater (∼ 30%) catalytic effectiveness than that of the currently used *cis*-[Pt(NH₃)₂Cl₂].

### Oxidation of thiols by copper (I) complexes

When simple salts of copper (II), such as CuCl₂ or CuBr₂, are placed in an aqueous solution, they are aquotated, accompanied by subsequent hydrolysis and formation of oligonuclear aquahydroxo-complexes of copper (II).

These aquahydroxo-complexes of copper (II) in aqueous solutions containing thiols are almost instantly reduced, forming thiolate complexes of copper (I) - Cu^{I}ₖ(SR)ₘ, where k = 2 to 14, m ≥ 3k. The composition and structure of Cu^{I}ₖ(SR)ₘ can have the most diverse nature, ranging from binuclear (k = 2) to 14-ring (k =14) coordination compounds; not uncommonly, mixtures of compounds of different structure are formed. However, no matter what the composition and structure of these complexes, they all effectively catalyse the processes of oxidation of -S-H groups into disulfide bridges -S-S- under the action of any given oxidizing agents, even very weak ones.

As an example, Figure 2 shows the results of an investigation of the relative catalytic effectiveness of Cu^{I}ₖ(SR)ₘ complexes in the reaction of oxidation of glutathione by hydrogen peroxide as compared to the binuclear palladium complex [Pd₂(µ-SG)₂(NH₃)₄]²⁺.

As can be seen from the results presented in Fig. 2, the Cu^{I}ₖ(SR)ₘ complexes act noticeably more effectively as catalysts of the oxidation process. However, aqueous solutions of glutathione disulfide containing Cu^{I}ₖ(SR)ₘ are unstable, according to ¹H NMR, IR spectroscopy and HELC data, and in aerobic conditions processes of a more thorough oxidation of the resulting disulfides begin in 30-60 minutes. This makes it practically impossible to use the Cu^{I}ₖ(SR)ₘ coordination compounds as selective catalysts of the oxidation of thiols to their disulfide forms.

### Oxidation of thiols with the use of Cu-Pd catalysts

Experimental studies of catalytic systems with the use of the copper-palladium catalysts [Pd₂^{II}(µ-SR)₂(NH₃)₄]·{Cu^{I}ₖ(SR)ₘ} demonstrate their much greater catalytic effectiveness as compared to the binuclear thiolate-bridged palladium (II) compounds [Pd₂^{II}(µ-SR)₂(NH₃)₄] (Fig. 3). But unlike aqueous solutions of oxidized glutathione GSSG containing thiolate complexes of copper Cu^{I}ₖ(SR)ₘ, aqueous solutions containing Pd-Cu catalysts are stable to processes of breakdown in cases where the concentration of the copper atoms does not exceed the concentration of palladium ions, according to the data of ¹H NMR, IR spectroscopy, and HELC. Surpassing the ratio of Pd:Cu by more than 1:1 in aerobic conditions is accompanied by a slow breakdown of GSSG. Thus, with a ratio Pd:Cu of only 1:2, the decrease in concentration of GSSG reaches a level of 96% in about 1 week.

Studies show that the Pd:Cu ratios in Pd-Cu catalysts lie in the range of 1:0.2 to 1:2, depending on the need to vary the working activity of the catalyst

Summarizing, for the processes of soft selective oxidation of thiols GSH to GSSG, it can be concluded that the binuclear thiolate-bridged complexes of palladium (II) play the major function of oxidation catalysts, while the thiolate complexes of copper (I) should be regarded as chemical sites that alter their catalytic activity or, in other words, control their catalytic activity.

Thus, the combining of functional binuclear palladium coordination compounds [Pd₂^{II}(µ-SR)₂(NH₃)₄] with bidentate bridge coordination of glutathione or acetylcysteine with a control site {Cu^{I}ₖ(SR)ₘ} formed from copper salts CuX₂ (where X= Cl⁻ or Br⁻) that are transformed in the medium of the thiols into corresponding thiolate derivatives of the copper (I) complexes shows us one of the most effective approaches to the control of the activity of catalytic systems based on palladium Pd₂^{II} and Cu^{I} complexes.

The increase in the catalytic effectiveness of the palladium-copper catalysts of general formula [Pd₂^{II}(µ-SR)₂(NH₃)₄]·{Cu^{I}ₖ(SR)ₘ} as compared to the functional chemical site [Pd₂^{II}(µ-SR)₂(NH₃)₄] is due to the fact that the hydrogen peroxide oxidizes not the Pd^{II} ions, but the {Cu^{I}ₖ(SR)ₘ} ions.

The chemical control site, forming {Cu^{II}ₖ(SR)ₘ} by the redox reaction

{Cu^{I}ₖ(SR)ₘ} + 2 H₂O₂ = {Cu^{II}ₖ(SR)ₘ} + 2 OH· + 2 OH⁻

plays the part of the oxidizing agent of the functional site [Pd₂^{II}(µ-SR)₂(NH₃)₄] according to scheme 2.

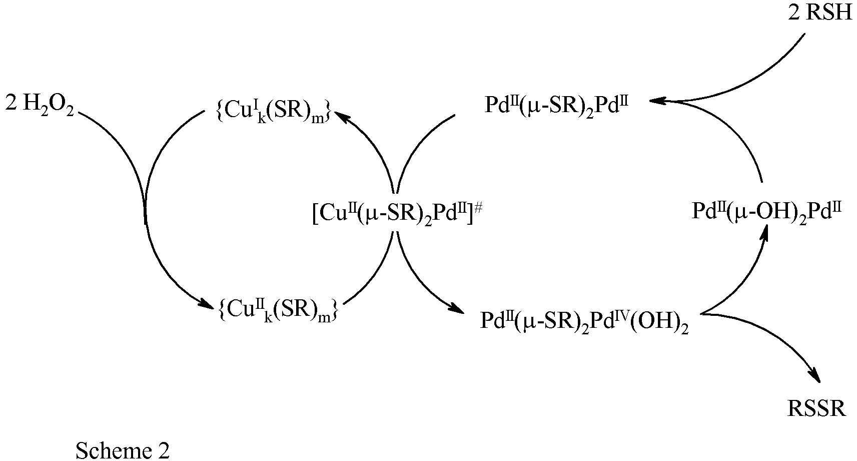

Two common types of transitional states exist for the redox reactions of coordination compounds of the d-elements, so-called *inside-sphere* and *outside-sphere* types. For the outside-sphere type, the coordination shells of the ions of the two metals do not touch each other. For the inside-sphere type, the ions of the two metals are bound by a bridge ligand, which is shared by both coordination shells.

The results of quantum chemistry calculations have shown that, in catalytic systems based on the binuclear metal skeleton Pd₂^{II}, an intermediate mixed Cu-Pd bimetallic center will be formed in aqueous solutions: [Cu^{II}(µ-SR)₂Pd^{II}]^{#} (an intermetallide), which is easily disproportionated into {Cu^{I}ₖ(SR)ₘ} and [Pd^{II}(µ-SR)Pd^{IV}(OH)₂].

Analysis of the structure of the outermost molecular orbitals (MO) explains the experimentally discovered faster reactivity of the catalytic system based on mixed Cu-Pd coordination compounds than that based on binuclear complexes containing only Pd^{II} ions.

The involvement of atomic d-orbitals (d-AO) of metals in catalytic processes removes the hindrances for the occurrence of the reaction stage by symmetry of molecular orbitals (MO) that often determine a high energy of activation of the processes. The main idea in explaining catalysis by metal ions for reactions that are forbidden by symmetry is that ions of the transitional metals have accessible d-AO lying close together in terms of energy. This allows molecules which coordinate to the metal ion to give up a pair of their electrons to certain d-orbitals of the metal and to receive them from other d-orbitals of the metal.

Analysis of the nature of the outermost MOs in a coordination compound with center Cu^{II}(µ-SR)₂Pd^{II} has shown that its highest occupied molecular orbital (HOMO) consists to a large extent of d-AO of copper and is not suitable to forming an oxidized product containing the ion Pd^{IV}. For the process to occur, it is necessary to transfer a pair of electrons to the lowest unoccupied molecular orbital (LUMO) with a large portion of 4d-orbitals of Pd^{II}. The smaller the difference in energies of these MOs, the less expenditure needed to achieve a transitional state. In the coordination compound under discussion with mixed bimetallic center Cu^{II}(µ-SR)₂Pd^{II} the energy difference between these orbitals is 2.61 eV. In the coordination compound with center Pd^{II}(µ-SR)₂Pd^{II} the difference in the energies of the corresponding occupied and unoccupied orbitals is much greater: 4.18 eV (for Pt^{II}(µ-SR)₂Pt^{II} it is 4.79 eV).

Thus, the catalytic system for the selective oxidation of thiols based on temporarily formed mixed complexes of Cu^{I} and Pd^{II} should have a greater activity than the system based on analogous Pd^{II} complexes. The reason for this is the level of energetics of the copper d-orbitals.

The formation of the intermediate bimetallic center [Cu^{II}(µ-SR)₂Pd^{II}]^{#} in the cycle accounts for the increasing of the catalytic effectiveness of the catalysts of general formula [Pd₂^{II}(µ-SR)₂(NH₃)₄] {Cu^{I}ₖ(SR)ₘ}, while an appropriately determined number of active bimetallic centers [Cu^{II}(µ-SR)₂Pd^{II}]^{#} enables a changing of the overall activity of palladium-copper catalysts.

The proposed catalysts of the invention can be coupled with N-glutamyl-L-cysteinyl-glycine and/or N-acetyl-L-cysteine disulfides, forming a combination having both natural biological and catalytic activity. An excess of thiol lets one prepare and utilize small and ultrasmall doses of the catalyst immediately, at the same time providing it with the substrate necessary for the catalytic cycle.

Free molecules of N-acetyl-cysteine and/or N-glutamyl-L-cysteinyl-glycine disulfides in the makeup of the preparation can be in either cationic or anionic form, or in the form of neutral molecules.

The counterion can be inorganic ions, such as cations of sodium, lithium, potassium, calcium, magnesium, selenium, manganese, zinc, vanadium and other chemical elements, or ions of organic compounds, such as amino acids, aliphatic and aromatic ions of organic molecules from various chemical groups having biological activity (example Nº 12).

The combinations according to the invention can also contain other pharmacologically active compounds, in particular purine and/or pyrimidine bases, their derivatives, or compounds based on them (examples Nº 10 and Nº 11).

By pharmacologically active compound is meant any substance that is used with therapeutic purposes, constituting molecules of medicinal and/or biologically active substances, in particular, purine and/or pyrimidine bases and compounds based on them, such as: Adenosine (9-β-D-ribofuranosyladenine), Guanosine (9-β-D-ribofuranosylguanidine), Desoxyadenosine (9-β-D-desoxyribofuranosyladenine), Desoxyguanosine (9-β-D-desoxyribofuranosylguanidine), 9-β-D-ribofuranosyladenine mono, di, triphosphate, 9-β-D- ribofuranosylguanidine mono, di, triphosphate, 9-β-D- desoxyribofuranosyladenine mono, di, triphosphate (cordycepin), 9-β-D-desoxyribofuranosylguanidine mono, di, triphosphate, Cytidine (4-a 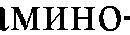 -1-[3,4-dihydroxy-5-(hydroxymethyl)tetrahydrofuran-2-yl]pyrimidin-2-one) . 1-[(2R,4S,5R)-4-hydroxy-5-(hydroxy)tetrahydrofuran-2-yl]-5-pyridine-2,4-dione, Desoxycytidine (4-amino-1-[4-hydroxy-5-(hydroxymethyl)tetrahydrofuran-2-yl]pyrimidin-2-one), Thymidine or desoxythymidine 1-[(2R,4S,5R)-4-hydroxy-5-(hydroxy)tetrahydrofuran-2-yl]-5-methylpyridine-2,4-dione, Inosine 9-[(2R,3R,4S,5R)-3,4-dihydroxy-5-(hydroxymethyl)oxolan-2-yl]-6,9-dihydro-3H-purin-6-one, Ribaverin: 1-(3,4-dihydroxy-5-hydroxymethyl-tetrahydrofuran-2-yl)-1H-[1,2,4]triazole-3-carboxylic acid amide, Zanamivir (2*R*,3*R*,4*S*)-4-[(diaminomethylidene)amino]-3-acetamido-2-[(1*R*,2*R*)-1,2,3-trihydroxypropyl]- 3,4-dihydro - 2*H*-pyrane- 6-carboxylic acid, Farmcyclovir: 2-[2-(2-amino-9H-purin-9-yl)ethyl]-1,3-propanediol diacetate (ester), Gancyclovir. (2-amino-1,9-dihydro-9-2-hydroxy-1-(hydroxymethyl)ethoxymethyl-6H-purin-6-one), Zidovudin (3'-azido-3'-desoxythymidine), Acyclovir: 2-amino-9-((2-hydroxyethoxy)methyl)-1H-purin-6(9H)-one, Fluoruracil: 5-fluoro-1H-pyrimidin-2,4-dione, 1-(2,3-didesoxy-beta-D-glyceropent-2-enofuranosyl) thymidine, Fluorothiouracil: 5-fluoro-1H-pyrimidine-2-thion-4-one, Thiouracil: 1H-pyrimidine-2-thion-4-one, S-adenosylhomocysteine: 4[5-(6-amino-purin-9-yl)-3,4-dihydroxy-tetrahydro-furan-2-ylmethylsulfanyl]-2-mercapto-butanoic acid, S-Adenosylmethionine: (2S)-2-amino-4-[[(2S,3S,4R,5R)-5-(6-aminopurin-9-yl)-3,4-dihydroxyoxolan-2-yl]methyl-methylsulfonio]butanoate, cyclical adenosine monophosphate (cAMP) 6-(6-amino-purin-9-yl)-2-oxo-tetrahydro-2λ-5-furo[3,2-d][1,2,3]d-isooxophosphinine-2,7diole, 8-chloro-cAMP: 6-(6-amino-8 chloro-purin-9-yl)-2-oxotetrahydro-2λ-5-furo[3,2-d][1,2,3]d-isooxophosphinine-2,7diole, N6,2'-O-dibutyryl-8-SH- cAMP, N6,2'-O-dibutyryl-8-SH-cAMP, cyclical guanosine monophosphate cGMP, N6-monobutyryl-8-S-methyl-cAMP, Formicin: 2-(7-amino-1P-pyrazolo [4,3-d] pyrimidin-3-yl)-5-hydroxymethyl-tetrahydrofuran-3,4-diol, -methylisoguanosine: 6-amino-1-methyl-1,9-dihydro-purin-2-one.

The pharmacologically active compound can be bound to an excess of the N-acetyl-cysteine and/or N-glutamyl-L-cysteinyl-glycine disulfides by van der Waals forces (ionic, hydrogen, and other noncovalent bonds).

The combinations according to the invention can be prepared by methods known to the art, taking into account the peculiarities of the chemical properties of the palladium-copper catalysts of the invention, from the disulfides (of N-acetyl-cysteine and/or N-glutamyl-L-cysteinyl-glycine) and the pharmacologically active substances. Preferably, the share of the catalyst according to the invention in the preparation is between 1·10⁻² and 1·10⁻⁷ g per mole of the disulfide of the aliphatic thiol - N-acetyl-cysteine and/or N-glutamyl-L-cysteinyl-glycine.

The palladium-copper catalyst proposed according to the invention or the catalytic combination of the invention can be used to strengthen the therapeutic activity of a purine and/or pyrimidine base or a derivative based on them. In the context of the present specification, the increase in the therapeutic effectiveness of the pharmacologically active compound is a lowering of the onetime or regimen dose, or a lowering of the overall toxicity and achievement of a more pronounced therapeutic effect given the usual therapeutic dose or less for this pharmacologically active compound.

The palladium-copper catalyst according to the invention, the catalytic combination and the pharmacological combination according to the invention can be used in the form of pharmaceutical compositions.

To obtain the pharmaceutical compositions according to the invention, one uses pharmaceutically acceptable excipients. In particular, these are inorganic or organic vehicles. Lactose, corn starch or its derivatives, talc, stearic acid or its salts and so forth can be used, for example, as such vehicles for tablets, shell-coated tablets, lozenges and hard gelatin capsules. Suitable vehicles for soft gelatin capsules are, for example, vegetable oils, waxes, fats, semisolid and liquid polyols, and so forth. Suitable vehicles for producing solutions and syrups are, for example, water, polyols, saccharose, invert sugar, glucose, and so forth. Suitable vehicles for suppositories are, for example, natural or solidified oils, waxes, fats, semiliquid or liquid polyols, and so forth.

Moreover, the pharmaceutical compositions can contain preservatives, solubilizers, stabilizers, flavoring agents, emulsifiers, sweeteners, colorants, correctors, salts for regulating the osmotic pressure, buffers, masking agents or antioxidants and other essential components.

The palladium-copper catalyst or catalytic combination according to the invention and the pharmacologically active substances whose effectiveness they intensify can be present both in the same dosage form or in separate dosage forms. Administering them in separate dosage forms can be done simultaneously (simultaneous taking of two solid dosage forms, such as tablets, simultaneous injection, especially in the same syringe) or consecutively, when the patient is given or administered first the one dosage form and then the other dosage form. The interval between administering is preferably not longer than 1 hour, although it can be increased up to the time when a synergistic effect is observed. The optimal administration sequence depends on the pharmacokinetics and pharmacodynamics of the pharmacologically active substance whose effectiveness is to be strengthened (rate of uptake, distribution, rate of elimination, features of the cell or organ tropics or systemic tropics) and it can be chosen individually for each particular substance.

The quantity of palladium-copper catalyst administered is determined by the mass share of Pd and Cu in the composition of the catalyst, which can be equal to or less than the daily requirement for each metal. Otherwise, the quantity of d-metal administered in the composition of the coordination compound is determined by the need to achieve a treatment result

The therapeutic result can be achieved by administering the catalyst in a quantity of 1·10⁻³ to 1·10⁻⁸ g per kg of body weight of the patient, which converting to the quantity of disulfide in the combination amounts to 1×10⁻² to 1×10⁻⁵ mole of disulfide per kg of body weight.

In general, all the products and methods according to the invention can alternatively include, consist of, or essentially consist of any suitable components and stages disclosed in the present specification or known to the skilled person from the prior art, and such products or methods according to the invention can additionally or alternatively exclude any given component, or stage, or object that is used in a product or method known from the prior art, or which is not essential to achieving the technical result of the present invention

### EXAMPLES

The invention will be explained below by specific examples.

### Example Nº 1. Producing the Pd-Cu catalyst on the basis of an ammoniated complex of palladium (II) with N-glutamyl-L-cysteinyl-glycine (GSH)

50 mg (237 mcmole) of cis-[Pd(NH₃)₂Cl₂] are placed in 10 ml of a solution containing 75 mg (244 mcmole) of GSH and homogenized in an ultrasound bath until a light yellow solution is formed. To the obtained system is poured in 5 ml of a solution containing 20 mg (117 mcmole) of CuCl₂·H₂O and the pH of the resulting light green solution is corrected to 5.0-5.2 with a solution of sodium hydroxide (∼ 0.01M).

The obtained solution of catalyst can be used to perform the oxidation of water-soluble thiols (e.g., GSH or acetylcysteine).

In the obtained solution of catalyst, the molar ratio of palladium to copper is 2:1.

### Example Nº 2. Producing the Pd-Cu catalyst on the basis of an ammoniated complex of palladium (II) with glutathione and its use for the synthesis of N-glutamyl-L-cysteinyl-glycine disulfide (GSSG).

### Stage 1. Producing an ammoniated complex of palladium (II) with glycylcysteinyl glutamate

10 mg (47.3 mcmole) of cis-[Pd(NH₃)₂Cl₂] is dispersed cold in 10-15 ml of distilled water, to the resulting suspension is added 145 mg (0.473 mmole) of GSH and mixing is done on a magnetic blender until a homogeneous light yellow solution is obtained

### Stage 2. Producing the Pd-Cu catalyst

To the previously obtained reaction mixture is poured in 5 ml of a solution containing 8.06 mg (47.3 mcmole) of copper (II) chloride dihydrate. The pH of the resulting yellowish-green catalyst solution is brought to a value of 5.5 - 5.8 with a 0.01M solution of sodium hydroxide.

### Stage 3. Using the Pd-Cu catalytic system for synthesis of GSSG

One weighs out into a glass 29.09 g (0.946 mmole) of GSH, pours in while mixing 150-200 ml of distilled water, cools to 10-15° C. Separately, one dissolves in 50-60 ml of distilled water 3.78 g of NaOH (0.946 mmole) and pours the resulting solution into the suspension of GSH under intense mixing and not letting the reaction mass heat up above 15-20° C, and mixes until obtaining a transparent homogeneous solution. The pH of the reaction mixture is corrected to 5.5-5.8 with a 0.1M solution of sodium hydroxide. To the obtained reaction system is poured in the previously prepared solution of catalyst, and 50 ml of a 1M freshly prepared solution of hydrogen peroxide is poured in by small portions with intense mixing, not allowing the reaction mixture to heat up beyond 15° C. The reaction is monitored for completion with HELC.

After completion of the reaction and sterilizing filtration, the solution is frozen and subjected to vacuum sublimation (lyophilic) drying.

The molar ratio of "sodium salt of N-glutamyl-L-cysteinyl-glycine disulfide - palladium - copper" in the obtained preparation is 1000 - 1 - 1.

### Example Nº 3. Producing the Pd-Cu catalyst on the basis of an ammoniated complex of palladium (II) with N-glutamyl-L-cysteinyl-glycine (GSH) and its use for obtaining glycylcysteinyl glutamate of disodium ribofuranosylhypoxantin.

### Stage 1. Producing an ammoniated complex of palladium (II) with GSH

20 mg (94.6 mcmole) of cis-[Pd(NH₃)₂Cl₂] is dispersed cold in 20 ml of distilled water, to the resulting suspension is added 30 mg (97.6 mcmole) of GSH and mixing is done on until a homogeneous light yellow solution is obtained

### Stage 2. Producing the Pd-Cu catalyst

To the previously obtained reaction mixture is poured in 5 ml of a solution containing 14.52 mg (85.2 mcmole) of copper (II) chloride dihydrate. The pH of the resulting yellowish-green catalyst solution is brought to a value of 5.5 - 6.0 with a 0.01M solution of sodium hydroxide.

### Stage 3. Using the Pd-Cu catalytic system to obtain N-glutamyl-L-cysteinyl-glycine of disodium ribofuranosylhypoxantin

One weighs out into a glass 58.19 g (0.189 mole) of GSH, pours in while mixing 200 ml of distilled water, cools to 10-15° C. Separately, one dissolves in 50-60 ml of distilled water 7.57 g of NaOH (0.189 mole) and pours the resulting solution into the suspension of GSH under intense mixing and not letting the reaction mass heat up above 15-20° C, and mixes until obtaining a transparent homogeneous solution. If necessary, the pH of the reaction mixture is corrected to 5.5-6.0 with a 0.1M solution of sodium hydroxide. To the obtained reaction system is poured in the previously prepared solution of catalyst, the glass is transferred to an ice bath (5-10° C), and 100-102 ml (∼ 0.1 mole) of a 1M freshly prepared solution of hydrogen peroxide is poured in by small portions with intense mixing over the course of 45-60 min. The reaction is monitored for completion by the HELCB method.

Separately, in 150 ml of hot distilled water (60-70° C) one dissolves 25.38 g (0.095 mole) of ribofuranosylhypoxantin (inosine). After complete dissolving, the solution is cooled to room temperature and poured into the reaction mixture. After sterilizing filtration, the solution is frozen and subjected to vacuum sublimation (lyophilic) drying

The molar ratio of GSSG - inosine - palladium - copper in the obtained preparation is 1000-1000-1-0.9.

### Example Nº 4. Producing the Pd-Cu catalyst on the basis of an ammoniated derivative of the binuclear coordination compound of palladium (II) with N-acetyl-L-cysteine.

120 mg (568 mcmole) of cis-[Pd(NH₃)₂Cl₂] are dispersed in an ultrasound bath in 20 ml of an aqueous solution of N-acetyl-L-cysteine (648.5 mg, 3.97 mmole) until a homogeneous yellow solution is formed. To the resulting system is poured in 5 ml of a solution containing 48.4 mg (284 mcmole) of CuCl₂·2H₂O. The white precipitate that settles out is dissolved while alkalinizing the reaction system to pH 4.5-5.0 by a 0.1M solution of sodium hydroxide.

The resulting greenish yellow solution of catalyst can be used to oxidize water-soluble thiols (such as reduced glutathione or acetylcysteine) or it can be lyophilized for later use

The molar ratio of the quantities of palladium and copper in the resulting solution of catalyst is 2:1.

### Example Nº 5. Producing the Pd-Cu catalyst on the basis of an ammoniated derivative of a binuclear coordination compound of Pd (II) with N-acetyl-L-cysteine and its use for the synthesis of the sodium salt of N-acetyl-L-cysteine disulfide.

### Stage 1. Producing an ammoniated complex of palladium (II) withN-acetyl-L-cysteine

16 mg (75.7 mcmole) of cis-[Pd(NH₃)₂Cl₂] is dispersed in an ultrasound bath in 20 ml of an aqueous solution of N-acetyl-L-cysteine (123.5 mg, 757 mcmole) until a homogeneous yellow solution is obtained.

### Stage 2. Producing the Pd-Cu catalyst

To the obtained solution is poured in 5 ml of a solution containing 6.5 mg (37.8 mcmole) of CuCl₂·2H₂O. The resulting catalyst solution is alkalinized to pH 4.5 - 5.0 with a saturated solution of lithium hydroxide

### Stage 3. Using the Pd-Cu catalytic system to obtain the sodium salt of N-acetyl-L-cysteine

In 250 ml of distilled water are dissolved 24.7 g (0.151 mole) of N-acetyl-L-cysteine, the solution of catalyst is poured in, and to the resulting solution is added, under intense mixing, 6.35 g (0.151 mole) of sodium hydroxide. After complete dissolving of the NaOH in H₂O, the pH of the solution is corrected to 5.5-6.0 by a saturated solution of sodium hydroxide and cooled to 10-15° C.

To the resulting solution in the cold state is added, in small portions and under intense mixing, a 1M solution of hydrogen peroxide (∼80 ml), not letting the temperature of the reaction mixture to rise above 15-20° C. The reaction is monitored for completion by the HELCB method.

After completion of the reaction and sterilizing filtration, the solution is frozen and subjected to vacuum sublimation (lyophilic) drying.

The molar ratio of "sodium salt of N-acetyl-L-cysteine disulfide - palladium - copper" in the obtained preparation is 1000 - 1 - 0.5.

### Example Nº 6. Producing the Pd-Cu catalyst on the basis of an ammoniated derivative of a binuclear coordination compound of Pd (II) with N-acetyl-L-cysteine and its use for the synthesis of the lithium salt ofN-acetyl-L-cysteine disulfide

### Stage 1. Producing an ammoniated complex of palladium (II) withN-acetyl-L-cysteine

16 mg (75.7 mcmole) of cis-[Pd(NH₃)₂Cl₂] is dispersed in an ultrasound bath in 20 ml of an aqueous solution of N-acetyl-L-cysteine (123.5 mg, 757 mcmole) until a homogeneous yellow solution is obtained.

### Stage 2. Producing the Pd-Cu catalyst

To the obtained solution is poured in 5 ml of a solution containing 6.5 mg (37.8 mcmole) of CuCl₂·2H₂O. The resulting catalyst solution is alkalinized to pH 4.5 - 5.0 with a saturated solution of lithium hydroxide

### Stage 3. Using the Pd-Cu catalytic system to obtain the lithium salt of N-acetyl-L-cysteine

In 250 ml of distilled water are dissolved 24.7 g (0.151 mole) of N-acetyl-L-cysteine, the solution of catalyst is poured in, and to the resulting solution is added, under intense mixing, 6.35 g (0.151 mole) of lithium hydroxide monohydrate. After complete dissolving of the LiOH·H₂O, the pH of the solution is corrected to 5.5-6.0 by a saturated solution of lithium hydroxide and cooled to 10-15° C.

To the resulting solution in the cold state is added, in small portions and under intense mixing, a 1M solution of hydrogen peroxide (∼80 ml), not letting the temperature of the reaction mixture rise above 15-20° C. The reaction is monitored for completion by the HELCB method.

After completion of the reaction and sterilizing filtration, the solution is frozen and subjected to vacuum sublimation (lyophilic) drying.

The molar ratio of "lithium salt of N-acetyl-L-cysteine disulfide - palladium - copper" in the obtained preparation is 1000 - 1 - 0.5.

### Example Nº 7. Producing the Pd-Cu catalyst on the basis of an ammoniated derivative of a binuclear coordination compound of Pd (II) with N-glutamyl-L-cysteinyl-glycine disulfide (GSH) and its use for the synthesis of the lithium salt of N-glutamyl-L-cysteinyl-glycine disulfide

### Stage 1. Producing an ammoniated complex of palladium (II) with GSH

21.1 mg (100 mcmole) of cis-[Pd(NH₃)₂Cl₂] is dispersed in an ultrasound bath in 20 ml of an aqueous solution of GSH (307.5 mg, 1 mmole) until a homogeneous yellow solution is obtained.

### Stage 2. Producing the Pd-Cu catalyst

To the obtained solution is poured in 5 ml of a solution containing 34.1 mg (200 mcmole) of CuCl₂·H₂O. The resulting catalyst solution is alkalinized to pH 5.0 - 5.5 with a saturated solution of lithium hydroxide

### Stage 3. Using the Pd-Cu catalytic system to obtain the lithium salt of N-glutamyl-L-cysteinyl-glycine disulfide

In 400-500 ml of distilled water are dissolved 61.5 g (0.2 mole) of GSH, the solution of catalyst is poured in, and to the resulting solution is added, under intense mixing, 8.39 g (0.2 mole) of lithium hydroxide monohydrate. After complete dissolving of the LiOH ·H₂O, the pH of the solution is corrected to 5.5-5.8 by a saturated solution of lithium hydroxide and cooled to 10-15° C.

To the resulting solution in the cold state is added, in small portions and under intense mixing, a 1M solution of hydrogen peroxide (∼110 ml), not letting the temperature of the reaction mixture rise above 15° C. The reaction is monitored for completion by the HELCB method.

After completion of the reaction and sterilizing filtration, the solution is frozen and subjected to vacuum sublimation (lyophilic) drying.

The molar ratio of lithium salt of N-glutamyl-L-cysteinyl-glycine disulfide - palladium-copper in the obtained preparation is 1000 - 1 - 2.

### Example Nº 8. Producing the catalyst on the basis of an ammoniated derivative of a binuclear coordination compound of Pd (II) with N-glutamyl-L-cysteinyl-glycine (GSH) and its use for the synthesis of the disulfide of glycylcysteinyl glutamate 1-[(2R,3R,4S,5R)-3,4-dihydroxy-5-(hydroxymethyl)oxolan-2-yl]-1H-1,2,4-triazolo-3-carboxyamide (GSSG-ribavirin)

### Stage 1. Producing an ammoniated complex of palladium (II) with GSH

20 mg (94.6 mcmole) of cis-[Pd(NH₃)₂Cl₂] is dispersed in an ultrasound bath in 20 ml of an aqueous solution of GSH (291 mg, 947 mcmole) until a homogeneous yellow solution is obtained.

### Stage 2. Producing the Pd-Cu catalyst

To the obtained solution is poured in 5 ml of a solution containing 24.2 mg (142 mcmole) of CuCl₂·H₂O. The resulting catalyst solution is alkalinized to pH 5.5 - 8.0 with a 0.01M solution of sodium hydroxide.

### Stage 3. Using the Pd-Cu catalytic system to obtain GSSG - ribavirin

In 400-500 ml of distilled water are dissolved 87.3 g (0.284 mole) of GSH, the solution of catalyst and a solution of 11.35 g (0.284 mole) of sodium hydroxide in 50 ml of water are poured in under intense mixing. The pH of the solution is corrected to 5.5-5.8 by a 0.1M solution of sodium hydroxide and cooled to 10-15° C.

To the resulting solution in the cold state is added, in small portions and under intense mixing, a 1M solution of hydrogen peroxide (∼150 ml), not letting the temperature of the reaction mixture rise above 15° C. The reaction is monitored for completion by the HELCB method.

Separately, in 100-150 ml of hot distilled water (60-70° C) one dissolves 34.65 g (0.142 mole) of ribavirin. After complete dissolving, the solution is cooled to room temperature and poured into the reaction mixture.

After completion of the reaction and sterilizing filtration, the solution is frozen and subjected to vacuum sublimation (lyophilic) drying.

The molar ratio of sodium salt of GSSG - ribavirin - palladium - copper in the obtained preparation is 1000 - 1000 - 1 - 1.5.

### Example Nº 9. Comparative evaluation of the biological activity and toxicity of a platinum compound and the palladium-copper catalyst when acting on cell growth.

Platinum compounds have pharmacological activity due to a catalytic action in reactions of oxidative modification of the sulfhydryl groups of molecules of a peptide nature, which lies at the heart of their stimulating action on the production of cytokines by the cellular effectors of the immune system, the selective inhibition of reactions of multiple drug resistance to antibiotics, and an ability to suppress the development of autoimmune reactions lying at the heart of many chronic and socially significant illnesses - psoriasis, neurodegenerative and viral diseases.

Thus, platinum compounds have a number of intrinsic properties that are sought after in pharmacological solutions and dictated by a catalytic action in reactions of oxidative modification of the sulfhydryl groups of molecules of peptide nature. However, platinum compounds have a high toxicity, whose mechanism is not related to the catalytic activity. The toxicity of platinum chemical compounds is of an acute kind, i.e., it appears rather quickly are a substance containing platinum is administered or gets into the body, once the maximum allowable concentration of platinum is exceeded. When platinum is administered in the makeup of substances below the maximum allowable concentration, a gradual buildup of platinum may occur in the tissues of various organs. In this case, the toxic action of platinum compounds appears later on or it does not appear at all with a characteristic poisoning pattern, but the mutagenic action of platinum can serve as a cause of developing malignant tumors.

The synthesized catalysts based on coordination compounds of aliphatic thiols (N-glutamyl-L-cysteinyl-glycine - GSH, N-acetyl-L-cysteine) and the d-metals palladium and copper (examples Nº 1, Nº 4) are also characterized by catalytic activity in the chemical reaction of oxidation of thiols in the makeup of molecules of a peptide nature and pharmacologically desirable thiol-containing molecules that is peculiar to coordination compounds of platinum. The palladium and copper compounds are not toxic, as compared to the platinum compounds, and have no intrinsic mutagenic or teratogenic action. Given that the catalytic activity of platinum compounds is related to pharmacologically desirable effects (including the chemotherapy of cancer illnesses, where the toxic action of platinum compounds is desirable), it was necessary to compare the biological effects of coordination compounds of the aliphatic thiols GSH, N-acetyl-L-cysteine, and the d-metals palladium and copper with the biological activity of platinum coordination compounds. A431 cells were chosen as the subject of exposure to the coordination compounds. Biochemical aspects of the action of platinum coordination compounds with aliphatic thiols have been investigated rather thoroughly in them. The nature of the cellular response allowed an evaluating of the similarity and/or difference in action on the cells of a catalyst based on coordination compounds of GSH and the d-metals palladium and copper, N-acetyl-L-cysteine and the d-metals palladium and copper, and oxidized glutathione and platinum

Goal of the work. To study the influence of a catalyst based on coordination compounds of the aliphatic thiols GSH, N-acetyl-L-cysteine, and the d-metals palladium and copper, and of a coordination compound of oxidized glutathione and platinum, on the growth of a cell culture.

The compounds investigated were a catalyst based on a coordination compound of the aliphatic thiol N-glutamyl-L-cysteinyl-glycine and the d-metals palladium and copper (compound Nº 1, synthesized in keeping with the description in example Nº 1 and Nº 2), a catalyst based on a coordination compound of N-acetyl-L-cysteine and the d-metals palladium and copper (compound Nº 2, synthesized in keeping with the description in example Nº 4, Nº 5), and a coordination compound of oxidized glutathione and cisplatin (compound Nº 3, synthesized in accordance with the technique described in the text of the patent 2153350).

### Preparation of the samples for the investigation.

The compounds being studied were kept at +4° C; immediately before the start of the experiment, the substances were dissolved in deionized water (super Q). The concentration of the initial solution exceeds, by a factor of 1000 or more times, the concentrations used in the experiment. The prepared concentrated solution is kept not longer than 5 hours at +4° C.

### Route of administration.

The compounds were added to the cell culture medium to the end concentration being studied.

### Number of doses.

In one of the series of experiments the preparations are added to the cells one time and the cells were incubated for 48 hours.

In another series for the indicated period of time of the experiments the preparations are added to the cells once a day for five times after every 24 hours and the cells were incubated for 120 hours. In control series, a corresponding volume of physiological solution was given.

### Concentrations.

All the compounds were added to a final concentration of 0.15 mcmole/ml (the concentration was calculated in terms of the content of excess aliphatic thiol disulfide);

### Criteria for evaluating the effect

The quantity of living and dead cells in the culture after 24 and 48 hours was determined in the first series of experiments and after 24, 48, 72, 96, 120 hours in the second series of experiments.

### Cell line used.

Cells of human epidermoid carcinoma of line A431, obtained from the All-Russian Collection of Cell Cultures (Institute of Cytology, Russian Academy of Sciences, St. Petersburg).

### Cell culturing conditions.

The cells were cultivated in a CO₂ incubator (New Brunswick Scientific) at +37° C and with 5% content of CO₂. The cells are grown under these conditions until a single layer of culture is formed and then they are subjected to the action of the compounds studied.

### Cell culture medium.

For the cell culturing we used medium DMEM (OOO "PanEko", Moscow) with an addition of gentamycin K (80 mg/l), L-glutamine (300 mg/l) and fetal serum (PAA, Austria) to a final concentration of 10%. One day before the start of the experiments, equal quantities of the cells (1000/2.5mcl-400x10⁻³/ml are transferred to a medium with reduced content of serum - 0.5 %.

### Cell growth dynamics.

The A431 cells were sown on Petri dishes (Nunc) in a concentration of 10000/cm², and one day after reaching 10-15% of a monolayer they were subjected to the action of the preparations being studied.

### Preparing the cells for staining.

The culture medium of the A431 cells was collected in test tubes (Falkon) for full analysis of the dead detached cells, while the cells in the Petri dishes were washed with PBS (which was combined with the collected medium) and treated with a 0.25% trypsin solution in Versen

(Paneko) for around 10 minutes at room temperature until the cells became detached. The cells were then suspended by pipetting with an automatic pipette and combined with the previously collected medium. Samples were centrifuged for 5 minutes at 400 g at room temperature, the supernatant was removed, and the sediment was resuspended in phosphate salt buffer PBS pH 7.4.

### Staining of the cells with propidium iodide

Propidium iodide was added to the cell suspension to a concentration of 50 mcg/ml, 5-10 min before measurement in a flow cytofluorimeter Bruker ACR 1000. This stain is able to penetrate the damaged cell membrane, and the stained cells are dead.

### Methods of statistical processing of the results.

The statistical processing of the obtained results was done on a personal computer with the help of the application program package "STATISTICA 6.0".

### Results of the influence of the studied compounds on the proliferative activity and death of the cells

According to the obtained data, all investigated compounds when administered once to the cell culture medium stimulate proliferative activity (table1).

**Table 1.**

| Influence of the studied compounds on the proliferative activity of A431 cells for a onetime exposure. | | | |
|---|---|---|---|
| Studied compound | Culture age (h) | Total cells (× 10³/2.5mcl) | Dead cells (%) |
| Control (no addition of studied compounds) | 0 | 1.0 | - |
| | 24 | 8.7 | 22 |
| | 48 | 15.1 | 29 |
| Compound Nº1 | 0 | 1.0 | - |
| | 24 | 11.7 | 11 |
| | 48 | 21.4 | 11 |
| Compound Nº2 | 0 | 1.0 | - |
| | 24 | 12.3 | 9 |
| | 48 | 22.1 | 12 |
| | 0 | 1.0 | - |
| | 24 | 10.8 | 14 |
| Compound Nº3 | 48 | 19.1 | 19 |

The findings testify that, in a onetime action of the studied compounds on the cell culture, there is a stimulation of the proliferative activity as compared to the control series. The discovered effect is comparable for all studied substances. By the criterion of number of dead cells, a decrease in their number by a factor of two or more is noted for the action of compounds Nº1 and Nº2 as compared to the control and somewhat less for the action of compound Nº3 (50-60%). The similarity of results for the response of the cell culture to the action of the different coordination compounds is one of the indications of a common mechanism of action and cellular response to it. The onetime quantities of metal administered are relatively small. If one considers that cisplatin is toxic to cells, obviously a onetime administration is not enough to notice an effect for the complex of aliphatic thiol and cisplatin, or cisplatin itself, which can be liberated upon breakdown of the coordination compound. In this regard, an experiment was organized with a 120 hour incubation of cells with five-time exposure of the cells to each studied compound once every 24 hours. The results are presented in table 2.

**Table 2**

| Influence of the studied compounds on the proliferative activity of A431 cells for a five-time exposure. | | | |
|---|---|---|---|
| Studied compound | Culture age (h) | Total cells (×10³/2.5mcl) | *Dead cells (%)* |
| Control (no addition of studied compounds) | 0 | 1.0 | - |
| | 24 | 8.9 | 22 |
| | 48 | 17.3 | 29 |
| | 72 | 31.4 | 32 |
| | 96 | 49.1 | 32 |
| | 120 | 58.6 | 35 |
| | | | |
| Compound Nº1 | 0 | 1.0 | - |
| | 24 | 10.1 | 11 |
| | 48 | 19.6 | 9 |
| | 72 | 39.4 | 9 |
| | 96 | 78.7 | 12 |
| | 120 | 94.2 | 14 |
| | | | |
| Compound Nº2 | 0 | 1.0 | - |
| | 24 | 11.6 | 7 |
| | 48 | 23.1 | 9 |
| | 72 | 44. | 8 |
| | 96 | 79.8 | 11 |
| | 120 | 102.1 | 11 |
| | | | |
| Compound Nº3 | 0 | 1.0 | - |
| | 24 | 10.8 | 12 |
| | 48 | 16.6 | 27 |
| | 72 | 24.8 | 38 |
| | 96 | 29.5 | 48 |
| | 120 | 32.5 | 67 |

The results of the experiments reveal a practically identical proliferative activity of the cells after one day, both in the control and upon exposure to all studied compounds. The previously discovered law of less cell death after one day for exposure to the studied compounds as compared to the control is also maintained. However, the action on the cells of the coordination compound glutathione disulfide with cisplatin on the second and following days leads to a drop in the proliferative activity of the culture and intensified cell death by nearly twofold the value of the analogous indicators in the control. On the other hand, the action on the cell cultures of compound Nº1 and compound Nº2 produced a practically linear daily increase in the number of cells and a relatively constant level of their death

The findings, taken together, indicate certain laws which, on the one hand, are determined by the activity of the studied compound as a whole, and on the other hand reflect the peculiarities of the action on the biological subject of the metals forming the coordination compounds. Such a similar dynamics of growth and death of the cells after 24 hours when exposed to all the studied coordination compounds indicates a similarity of the mechanism of their action, regardless of the metal and the aliphatic thiol. However, the coordination compounds are not removed from the culture medium, and this means that every subsequent administration increases their concentration. If we consider the fact that the ligand is an actively metabolizable structure, the liberation of the metal complex is a logical assumption. Cisplatin is a stable molecule. Being nontoxic in the makeup of the complex compound, cisplatin manifests its cytotoxic biological action upon degradation of the ligand. In this regard, we observe an active increase in dead cells and a low proliferative activity. It is not ruled out that, in the closed system, not only cisplatin but also cisplatin-modified nucleotides have a cytotoxic action when they participate in the synthesis reactions of nucleic acids.

The biological activity of the d-metals palladium and copper is different from cisplatin. Palladium and its compounds are a relatively neutral molecule, biologically speaking. Copper is a bioelement and is actively used by the cells in the composition of various enzymes participating in reactions of energy liberation, detoxification, physiologically regulated synthesis and breakdown of biomolecules. In this regard, biologically positive effects of the catalyst based on the coordination compounds of aliphatic thiols of the d-metals palladium and copper were found in the conditions of the experiment.

**Conclusion.** Thus, catalysts based on coordination compounds of aliphatic thiols N-glutamyl-L-cysteinyl-glycine and N-acetyl-L-cysteine with the d-metals palladium and copper are characterized by a similar mechanism of action on cells, peculiar to the coordination compound of oxidized glutathione and cisplatin, however they lack the toxicity intrinsic to the coordination. In this connection, catalysts based on, complex compounds of aliphatic thiols of coordination compounds of aliphatic thiols N-glutamyl-L-cysteinyl-glycine and N-acetyl-L-cysteine with the d-metals palladium and copper can be used in pharmacological solutions with pharmaceuticals for therapy of varying length that requires a catalytic activity in reactions of oxidative modification of thiols to form disulfides in the composition of molecules of peptide nature.

### Example No. 10 Comparative evaluation of the ability of a coordination compound of oxidized glutathione and cisplatin and the ability of a catalyst based on a coordination compound of N-glutamyl-L-cysteinyl-glycine, palladium and copper, to potentiate the antiviral activity of inosine

The patents RU2153350 and RU2153351 discuss a pharmacological solution for potentiating the antiviral activity of inosine, preferably with the use of a coordination compound of cisplatin and oxidized glutathione. The potentiation effect is achieved preferably by virtue of the ability of the compound cisplatin and oxidized glutathione to catalyse a complex of reactions of oxidative modification of a target, enhancing its affinity for the action of inosine. If the mechanism of potentiating the antiviral effect of inosine is related to the catalytic activity of the coordination compounds, then the agent according to the invention should have a similar more pronounced action, since they are distinguished by a higher catalytic activity as compared to the coordination compound of cisplatin and oxidized glutathione.

**Goal of the study:** comparative evaluation of the antiviral activity of inosine in combination with a coordination compound of oxidized glutathione and cisplatin and a catalyst based on the coordination compound of N-glutamyl-L-cysteinyl-glycine palladium and copper.

### The compounds studied

Pharmacological composition (PC) Nº1 - N-glutamyl-L-cysteinyl-glycine of disodium ribofuranosylhypoxantin, containing the agent according to the invention (synthesis presented in example Nº3)

Pharmacological composition (PC) Nº2 - N-glutamyl-L-cysteinyl-glycine of disodium ribofuranosylhypoxantin, containing a coordination compound of cisplatin and oxidized glutathione (synthesis according to the technique presented in patents 2153350, 2153351)

### Experimental model

- *the virus of Venezuelan equine encephalitis (VEE) -* pathogen strain Trinidad. The accumulation of virus-containing material for subsequent infection of the laboratory animals was done using 9-11 day-old chick embryos - 30-50 of them. First of all, five consecutive tenfold dilutions of the virus-containing suspension were prepared. For each dilution of virus-containing suspension, 0.2 ml was introduced into the allantoic fluid of the developing chick embryos. The injection site of virus-containing suspension was covered with melted paraffin. Next, the developing chick embryos are placed in a thermostat at a temperature of (37±0,5)° C for 18 h, periodically checking their viability by means of an ovoscope. After the incubation time was over, the viability of the developing chick embryos was evaluated in the thermostat and from the "carcasses" of the living embryos a 10% suspension of virus-containing material was prepared, using physiological solution with an addition of antibiotics (penicillin, calculating 100 units per 1 ml, streptomycin 200 units per 1 ml). The resulting suspension was centrifuged for 10 min at 1.5-2.0 thousand rpm and temperature of plus (3±0.5)° C. The supernatant liquid was decanted into vials with a volume of 1.0 ml and used for the later infecting of the experimental animals, mice. The initial virus titer was 10⁷-10⁸ LD₅₀/ml
- *the virus of Rift valley fever (RVF) -* pathogenic strain 8-87. The accumulation of virus-containing material for infection of the laboratory animals is done using 3-5 day-old mouse pups, 10-15 of them. At first, five consecutive tenfold dilutions of the virus-containing material were prepared. For each dilution, 0.02 ml was introduced into the brain of the mouse pups and they were placed under observation for 24-48 h, after which the animals were sacrificed using ether, the cerebrum was extracted and placed, three specimens at a time, in penicillin vials which were kept in a freezer at temperature of minus (20±0.5)° C. After this, a 10% suspension of the cerebrum was used as the virus-containing material. The initial virus titer was 10⁵-10⁶ LD₅₀/ml;
- the tick-borne encephalitis (TBE) virus - pathogenic strain Absetarov. The accumulation of virus-containing material for infection of the laboratory animals was done in mouse pups. At first, five consecutive tenfold dilutions of the virus-containing material were prepared, using the centrifugate of a 10% suspension of the brain of previously infected mice or virus-containing material rehydrated from the lyophilized state. For each dilution, 0.02 ml was introduced into the brain of the mouse pups and they were placed under observation for 24-48 h, after which the animals were sacrificed using ether, the cerebrum was extracted and placed, three specimens at a time, in penicillin vials which were kept in a freezer at temperature of minus (20±0.5)° C. After this, a 10% suspension of the cerebrum was used as the virus-containing material. The initial virus titer was 10²-10³ LD₅₀/ml.

The studies evaluating the effectiveness of pharmacological compositions Nº1 and Nº2 were done on white non-inbred male mice weighing 16-18 g (360 mice).

The study made use of mice having spent a one week quarantine at the clinic of experimental biological models of the NIITs (MBZ) FGU "GosNIII VM of the Russian Ministry of Defense".

For each pathogen, we determined the LD₅₀ on white non-inbred mice, calculating this criterion by the Kerber method in the modification of I. P. Ashmarin and A. A. Vorob'yev.

The effectiveness of the studied preparations was determined by comparing the survival rates of the animals in the experimental groups (having received the corresponding preparations) and control groups. The percentage of surviving animals in the experimental and control groups was determined from the tables of Genes V.S. The observation of infected animals was done for 21 days, recording each day the number of living and deceased animals in the experimental and control groups.

### Methods of statistical processing of the results

The statistical processing of the results of the experiments was done on a personal computer, using special programs carrying out the traditional statistical methods.

### Results from the study of the antiviral activity of pharmacological compositions Nº1 and Nº2

In all the experimental models of the OOVI, the effectiveness of pharmacological compositions Nº1 and Nº2 was evaluated using two schemes:
- 24 h before infection, at the same time as infection, 24 h, 48 h and 72 h after infection (scheme 1 - emergency prophylaxis);
- at the same time as infection, 24 h, 48 h and 72 h after infection (scheme 2 - early etiotropic treatment).

Pharmacological compositions Nº1 and Nº2 were administered subcutaneously in a volume of 0.5 ml in a onetime dose of 30 mg/kg of body weight (10 mcg/mouse).

Effectiveness of pharmacological compositions Nº1 and Nº2 in experimental infection with VEE.

The results of the investigations are presented in table 3.

**Table 3**

| Comparative evaluation of the antiviral effectiveness of pharmacological compositions Nº1 and Nº2 as means of emergency prophylaxis and etiotropic treatment of experimental VEE infection | | | | | |
|---|---|---|---|---|---|
| Preparation | Scheme of admin., Nº | Infecting dose of pathogen (qty LD₅₀) | Number of animals in group | Number of deceased animals, % | Number of surviving animals, % |
| PC Nº1 | 1 | 12 | 10 | 0 | 100* |
| | | 12 | 10 | 0 | 100* |
| | 2 | 12 | 10 | 0 | 100* |
| | | 12 | 10 | 0 | 100* |
| PC Nº2 | 1 | 12 | 10 | 60 | 40 |
| | | 12 | 10 | 40 | 60 |
| | 2 | 12 | 10 | 50 | 50 |
| | | 12 | 10 | 40 | 60 |
| | | | | | |
| Infection control | Not done | 12 | 10 | 100 | 0 |
| | Not done | 12 | 10 | 100 | 0 |
| * - differences from the control parameters are reliable for p<0.05. | | | | | |

The data presented in table 3 testify that the evaluated preparations exerted a protective action in relation to experimental VEE. The most effective in the given conditions turned out to be PM Nº1. If the preparation was used by scheme 1 (24 h before infection, at the same time as infection, 24 h, 48 h and 72 h after infection), regardless of the infecting dose of VEE virus the survival rate of the infected mice was at a level of 100%, against a 100% lethality in the control. But if the preparation was used by scheme 2 (at the same time as infection, 24 h, 48 h and 72 h after infection), then in these conditions depending on the infecting dose of pathogen the protective effect was on a level of 100% (when infected by pathogen in a dose of 12 LD₅₀) and 100 % (when infected by pathogen in a dose of 2 LD₅₀). When using PC Nº2, the protection indicators depending on the infecting dose of pathogen were 40-60% lower than when using PC Nº1.

Thus, on the basis of the studies performed, it may be concluded that the most effective of the evaluated preparations in regard to experimental VEE infection was PC Nº1, which includes the agent according to the patent. PC Nº1, used in the emergency prophylaxis scheme, provided 100% protection of the infected animals, against 100% lethality in the control.

### Effectiveness of pharmacological compositions Nº1 and Nº2 in experimental RVF viral infection.

The results of the investigations are presented in table 4.

**Table 4**

| Comparative evaluation of the antiviral effectiveness of pharmacological compositions Nº1 and Nº2 as means of emergency prophylaxis and etiotropic treatment of experimental RVF | | | | | |
|---|---|---|---|---|---|
| Preparation | Scheme of admin., Nº | Infecting dose of pathogen (qty LD₅₀) | Number of animals in group | Number of deceased animals, % | Number of surviving animals, % |
| PC Nº1 | 1 | 12 | 10 | 0 | 100* |
| | | 12 | 10 | 0 | 100* |
| | 2 | 12 | 10 | 0 | 100* |
| | | 12 | 10 | 0 | 100* |
| PC Nº2 | 1 | 12 | 10 | 40 | 60 |
| | | 12 | 10 | 40 | 60 |
| | 2 | 12 | 10 | 70 | 30 |
| Infection control | Not done | 12 | 10 | 100 | 0 |
| | Not done | 12 | 10 | 100 | 0 |
| * - differences from the control parameters are reliable for p<0.05. | | | | | |

The data presented in table 4 testify that the best protective effect in relation to RVF was obtained by using PC Nº1. Regardless of the scheme used, the preparation afforded 100% protection of the infected mice, as against 100% lethality in the control. Pharmacological composition Nº2 was practically ineffective in the given conditions.

### Effectiveness of pharmacological compositions Nº1 and Nº2 in experimental viral infection, - experimental tick-borne encephalitis.

The results of the investigations are presented in table 5.

**Table 5**

| Comparative evaluation of the antiviral effectiveness of pharmacological compositions Nº1 and Nº2 as means of emergency prophylaxis and etiotropic treatment of experimental infection, experimental tick-borne encephalitis | | | | | |
|---|---|---|---|---|---|
| Preparation | Scheme of admin., Nº | Infecting dose of pathogen (qty LD₅₀) | Number of animals in group | Number of deceased animals, % | Number of surviving animals, % |
| PC Nº1 | 1 | 12 | 10 | 0 | 100* |
| | | 12 | 10 | 0 | 100* |
| | 2 | 12 | 10 | 0 | 100* |
| | | 12 | 10 | 0 | 100* |
| PC Nº2 | 1 | 12 | 10 | 20 | 80* |
| | | 12 | 10 | 0 | 100* |
| | 2 | 12 | 10 | 20 | 80* |
| | | 12 | 10 | 20 | 80* |
| | | | | | |
| | | | | | |
| | | | | | |
| Infection control | Not done | 12 | 10 | 100 | 0 |
| | Not done | 12 | 10 | 100 | 0 |
| * - differences from the control parameters are reliable for p<0.05. | | | | | |

As follows from the presented data, all the evaluated preparations when used in animals infected with the pathogen in a dose of 2 LD₅₀ exhibited practically the identical protective effectiveness, ensuring a survival rate of 80-100% of the infected mice, as against their 100% lethality in the control. At the same time, if the preparations were used in animals infected with the pathogen in a dose of 12 LD₅₀, PC Nº1 proved to be more effective than PC Nº2 in these conditions.

### Conclusion

The results of the studies conducted, taken altogether, allow us to conclude that pharmacological compositions Nº1 and Nº2 have an antiviral activity. However, PC Nº1, which includes the agent according to the invention, had a more pronounced antiviral activity as compared to pharmacological composition Nº2, which included the coordination compound of oxidized glutathione and cisplatin.

### Example 11. Determination of the ability of the agent according to the invention to potentiate the antiviral activity of ribavirin

Ribavirin is a specific antiviral product. Its action on virus-infected cells is similar to inosine. The agent according to the invention, able to stimulate the processes of oxidative modification of proteins, potentiated the antiviral effect of inosine. The similarity in the antiviral action of inosine and ribavirin lets us postulate the possibility of potentiating the antiviral effect of 1-[(2*R*,3*R*,4*S*,5*R*)-3,4-dihydroxy-5-(hydroxymethyl)oxolan-2-yl]-1*H*-1,2,4-triazole-3-carboxamide (the effective principle of the pharmacopoeia product Ribavirin^{®} ) by the agent according to the invention.

**Goal of the study:** to assess the ability of the agent according to the invention to potentiate the antiviral activity of 1-[(2*R*,3*R*,4*S*,5*R*)-3,4-dihydroxy-5-(hydroxymethyl)oxolan]-1*H*-1,2,4-triazole-3-carboxamide.

### The studied compounds

The pharmacological composition 1-[(2*R*,3*R*,4*S*,5*R*)-3,4-dihydroxy-5-(hydroxymethyl)oxolan-2-yl]-1*H*-1,2,4-triazole-3-carboxamide coupled with the agent according to the invention (synthesis presented in example Nº8) - preparation Nº1.

The pharmacopoeia antiviral product Ribavirin^{®} produced by "Kanonfarma Prodakshn" (Russia) - preparation Nº2.

### Experimental model

The studies were done on the experimental models described in example Nº10. The infecting dose of pathogen in each model corresponded to 10 LD₅₀.

### Results from the study of the antiviral activity of preparations Nº1 and Nº2.

On all experimental models of the OOVI, the effectiveness of preparations Nº1 and Nº2 was evaluated by using them in two schemes:
- 24 h before infection, at the same time as infection, 24 h, 48 h and 72 h after infection (scheme 1 - emergency prophylaxis);
- at the same time as infection, 24 h, 48 h and 72 h after infection (scheme 2 - early etiotropic treatment).

Preparations Nº1 and Nº2 were administered subcutaneously in a volume of 0.5 ml

Preparation Nº1 in a onetime dose of 30 mg/kg of body weight (10 mcg/mouse).

Preparation Nº2 in a onetime dose of 30 mg/kg of body weight (10 mcg/mouse)

Effectiveness of preparations Nº1 and Nº2 in experimental virus infection - Venezuelan equine encephalitis (VEE).

The results of the investigations are presented in table 6.

**Table 6**

| Effectiveness of preparations Nº1 and Nº2 in experimental virus infection - Venezuelan equine encephalitis (VEE) | | | | | |
|---|---|---|---|---|---|
| Preparation | Scheme of admin., Nº | Infecting dose of pathogen (qty LD₅₀) | Number of animals in group | Number of deceased animals, % | Number of surviving animals, % |
| Preparation Nº1 | 1 | 10 | 10 | 20 (2-56) | 80 (49-84)* |
| | 2 | 10 | 10 | 20 (2-56) | 80 (49-84)* |
| Preparation Nº1 | 1 | 10 | 10 | 50 (19-64) | 50 (19-64) |
| | 2 | 10 | 10 | 50 (19-64) | 50 (19-64) |
| Infection control | 1 | 10 | 10 | 100 (86-100) | 0 (0-31) |
| | 2 | 10 | 10 | 100 (86-100) | 0 (0-31) |
| * - differences from the control parameters are reliable for p<0.05. | | | | | |

The data presented in table 6 testify that the evaluated preparations differed from each other in terms of the protective effect forming when introduced into the body in relation to the experimental infection with VEE.

Preparation Nº1 ensured a survival rate of the infected mice on a level of 80%, as against 100% lethality in the control (p<0.05) for both schemes of administration, whereas the analogous effects of preparation Nº2 (ribavirin) did not exceed 50%.

Thus, on the basis of the studies carried out, we may conclude that preparation Nº1, being the active principle of the pharmacopoeia product Ribavirin^{®} coupled with the agent according to the invention, surpassed the action of the pharmacopoeia product Ribavirin^{®} in terms of protective and therapeutic effectiveness in regard to experimental infection with VEE by a factor of 1.5-2.0 times.

### Effectiveness of preparations Nº1 and Nº2 in experimental virus infection - Rift Valley fever (RVF)

The results of the investigations are presented in table 7.

**Table 7**

| Effectiveness of preparations Nº1 and Nº2 in experimental virus infection - Rift Valley fever (RVF) | | | | | |
|---|---|---|---|---|---|
| Preparation | Scheme of admin., Nº | Infecting dose of pathogen (qty LD₅₀) | Number of animals in group | Number of deceased animals, % | Number of surviving animals, % |
| Preparation Nº1 | 1 | 10 | 10 | 30 (19-64) | 70 (35-93)* |
| | 2 | 10 | 10 | 30 (19-64) | 70 (35-93)* |
| Preparation Nº2 | 1 | 10 | 10 | 70 (35-93) | 30 (19-64) |
| | 2 | 10 | 10 | 70 (35-93) | 30 (19-64) |
| Infection control | Not done | 10 | 10 | 100 (86-100) | 0 (0-31) |
| | Not done | 10 | 10 | 100 (86-100) | 0 (0-31) |
| * - differences from the control parameters are reliable for p<0.05. | | | | | |

The data presented in table 7 testify that the protective and therapeutic effect of preparation No. 1 in relation to experimental infection with RVF was manifested to a greater degree than that of preparation No. 2. In particular, the protective effect of preparation No. 1 was 70% as against 30% for preparation No. 2 and 100% lethality in the control.

Thus, on the basis of the studies carried out, we may conclude that preparation Nº1, being the active principle of the pharmacopoeia product Ribavirin^{®} coupled with the agent according to the invention, surpassed the action of the pharmacopoeia product Ribavirin^{®} in terms of protective and therapeutic effectiveness in regard to experimental viral infection by a factor of more than 2 times.

Effectiveness of preparations Nº1 and Nº2 in experimental virus infection - tick-borne encephalitis (TBE)

The results of the investigations are presented in table 8.

**Table 8**

| Effectiveness of preparations Nº1 and Nº2 in experimental virus infection - tick-borne encephalitis (TBE) | | | | | |
|---|---|---|---|---|---|
| Preparation | Scheme of admin., Nº | Infecting dose of pathogen (qty LD₅₀) | Number of animals in group | Number of deceased animals, % | Number of surviving animals, % |
| Preparation Nº1 | 1 | 10 | 10 | 20 (2-56) | 80 (44-98)* |
| | 2 | 10 | 10 | 30 (19-64) | 70 (35-93)* |
| Preparation Nº2 | 1 | 10 | 10 | 50 (19-81) | 50 (19-81) |
| | 2 | 10 | 10 | 50 (19-81) | 50 (19-81) |
| Infection control | Not done | 10 | 10 | 100 (86-100) | 0 (0-31) |
| | Not done | 10 | 10 | 100 (86-100) | 0 (0-31) |
| * - differences from the control parameters are reliable for p<0.05. | | | | | |

As follows from the data presented, preparation No. 1 when used in animals infected by the pathogen in a dose of 10 LD₅₀, regardless of the scheme used, exhibited a practically identical protective effectiveness, ensuring a survival rate of 70-80% of the infected mice, as against their 100% lethality in the control. At the same time, if preparation No. 2 was used in the infected animals, the effectiveness proved to be less pronounced. The preparation afforded a level of protection of 50%, regardless of the scheme used for its administration, which was 10-30% lower than that of preparation No. 1.

Thus, on the basis of the studies carried out, we may conclude that preparation Nº1, being the active principle of the pharmacopoeia product Ribavirin^{®} coupled with the agent according to the invention, surpassed the action of the pharmacopoeia product Ribavirin^{®} in terms of protective and therapeutic effectiveness in regard to experimental viral infection by a factor of 1.5-1.8 times.

### Conclusion

The results of the studies carried out, taken altogether, let us conclude that the agent according to the invention potentiated the antiviral action of 1-[(2*R*,3*R*,4*S*,5*R*)-3,4-dihydroxy-5-(hydroxymethyl)oxolan-2-yl]-1*H*-1,2,4-triazole-3-carboxamide, the active principle of the pharmacopoeia product Ribavirin^{®}. In this regard, the use of the agent according to the invention can be promising in the pharmacological solutions to create a new generation of drugs for the treatment and prevention of viral diseases of man and animals.

### Example 12. Influence of the agent according to the invention on the hemostimulating activity of lithium ions given in the form of the lithium salt of N-acetyl-L-cysteine disulfide.

At present, there is no effective hemostimulating agent that can be kept in lengthy storage. In medical practice, the hemostimulating activity of lithium in concentrations is known, being associated with the negative influence of lithium on the functions of the central nervous system

Goal of the investigation. To determine the ability of the agent according to the invention to potentiate the hemostimulating action of lithium ions.

### Preparation of the solutions of studied compounds for the investigation

The crystalline substances were kept at 4° C; immediately before the start of the experiment, the substance was dissolved in physiological solution. The solution was sterilized by being passed through filters of 0.22 mcm, Millex-GS (Millipore), in a sterile laminar flow box.

### Model of the investigation.

The investigation was performed on white random-breed male rats weighing 140-160 g, given a onetime dose of cyclophosphan (CPh) of 120 mg/kg subcutaneously in physiological solution.

Four groups of experimental animals were formed
Nº1 - intact animals having received injections of the solvent of the studied compounds (physiological solution) (solvent control);
Nº2 - animals having received an injection of CPh, afterwards being administered physiological solution as the treatment agent (control);

### Experiment groups:

Nº3 - animals having received an injection of cyclophosphan, afterwards being administered pharmacological composition No. 1 as the treatment agent (PC No. 1 was synthesized according to the description in example No. 6), containing lithium ions, N-acetyl-L-cysteine disulfide, and the agent according to the invention in physiological solution in a dose of 10 mg/kg (quantity of coordination compound 7.8×10⁻⁸M/kg);
Nº4 - animals having received an injection of cyclophosphan, afterwards being administered aC-Li as the treatment agent (preparation No. 2) in physiological solution in a dose of 10 mg/kg;
Nº5 - animals having received an injection of cyclophosphan, afterwards being administered lithium carbonate as the treatment agent (preparation No. 3) in physiological solution in a dose of 3 mg/kg (quantity of lithium carbonate calculated on the basis of the mass fraction of the lithium ion (0.55) in 10 mg of the lithium salt of cysteine, a similar quantity of lithium is contained in 3 mg of lithium carbonate);
Nº6 - animals having received an injection of cyclophosphan, afterwards being administered acetyl cysteine disulfide as the treatment agent (preparation No. 4) in physiological solution in a dose of 9.5 mg/kg;
The studied preparations were administered on day three after the administration of the cyclophosphan.

### Investigated material,

Blood was taken for the hematological studies from the caudal vein, 3, 7, and 14 days after administration of CPh. At the end of each series (3, 7 and 14 days), the experimental animals were euthanized by ether overdose and blood was taken from the caudal vein and bone marrow of the femur.

### Analyzed indicators.

In the present investigation, we evaluated the blood cell pattern (number of erythrocytes, thrombocytes, leucocytes, lymphocytes and neutrophils, and also the ESR) and bone marrow for a 7-day administration of the studied compounds to hemodepressed animals.

### Results of the investigation

The results of the investigations of the blood pattern are presented in tables 9-11.

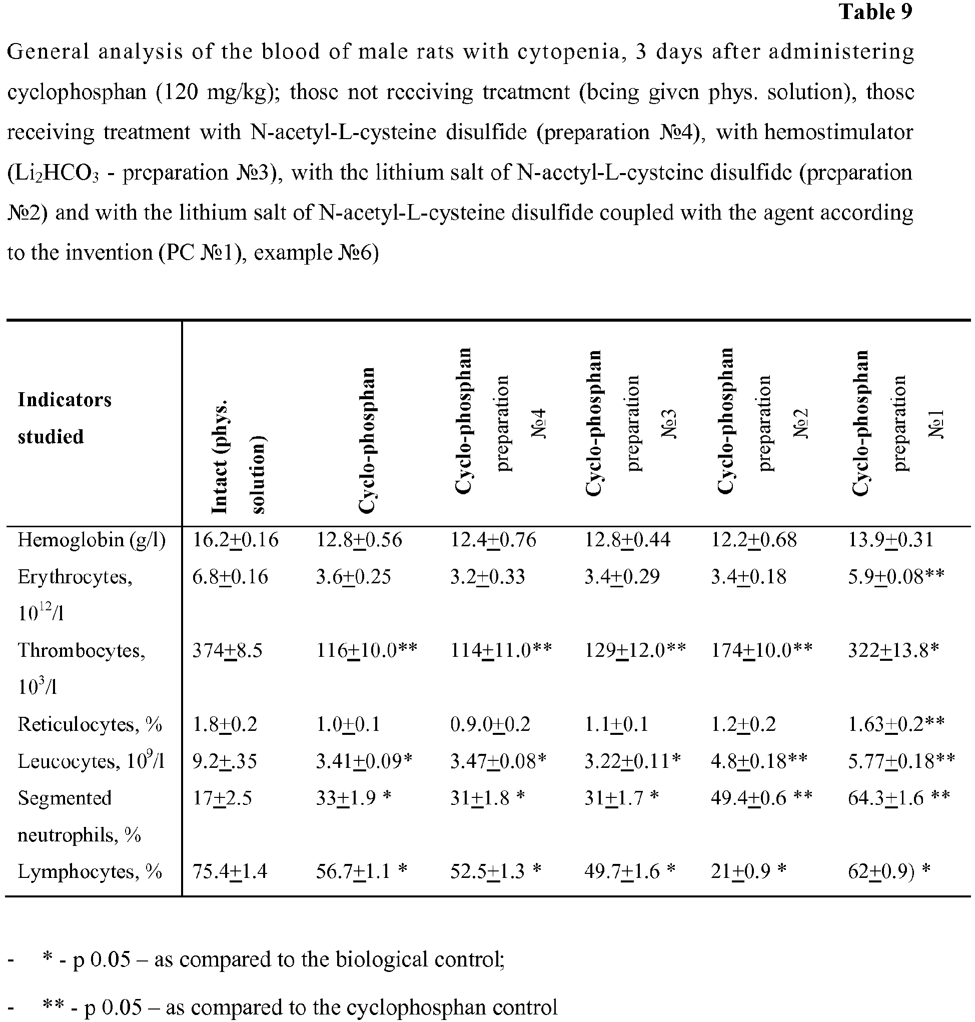

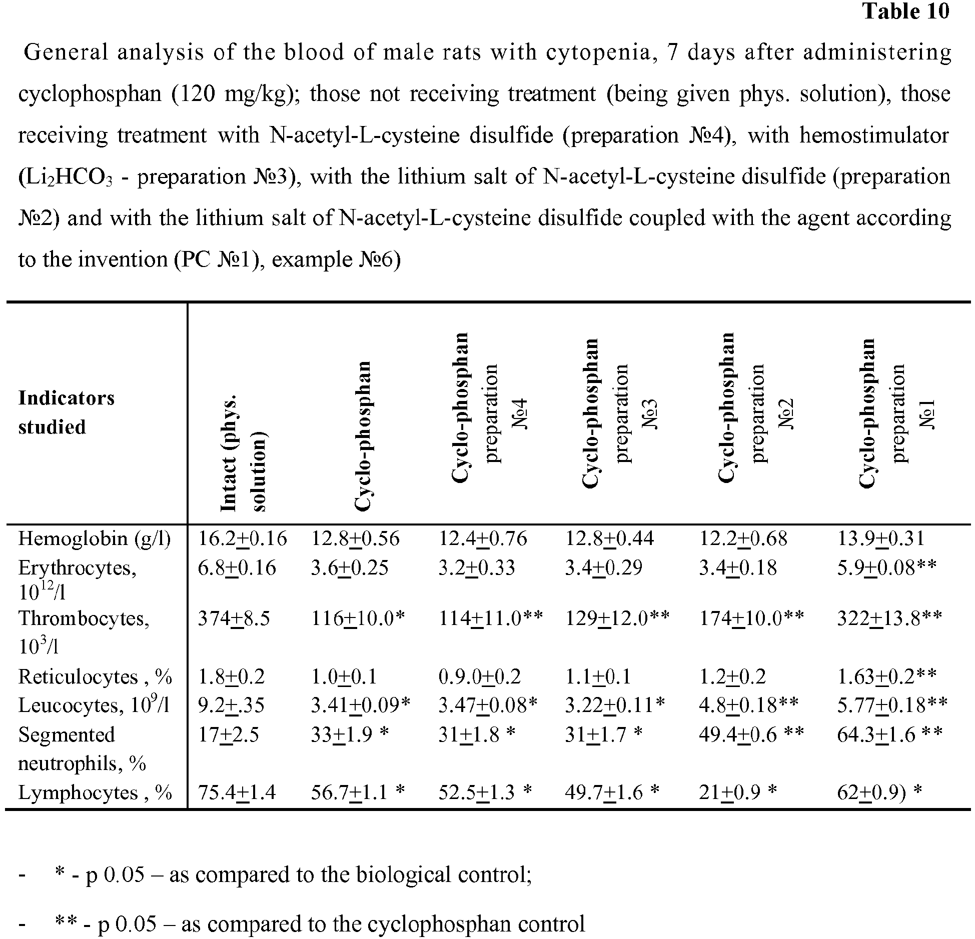

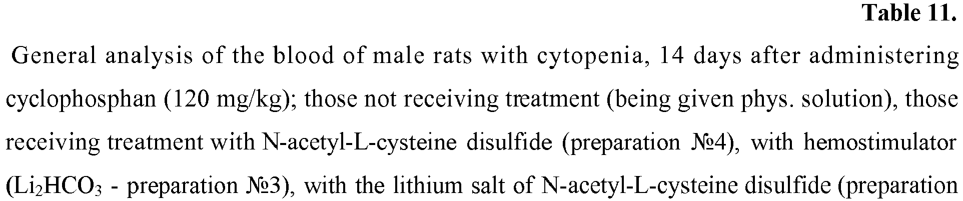

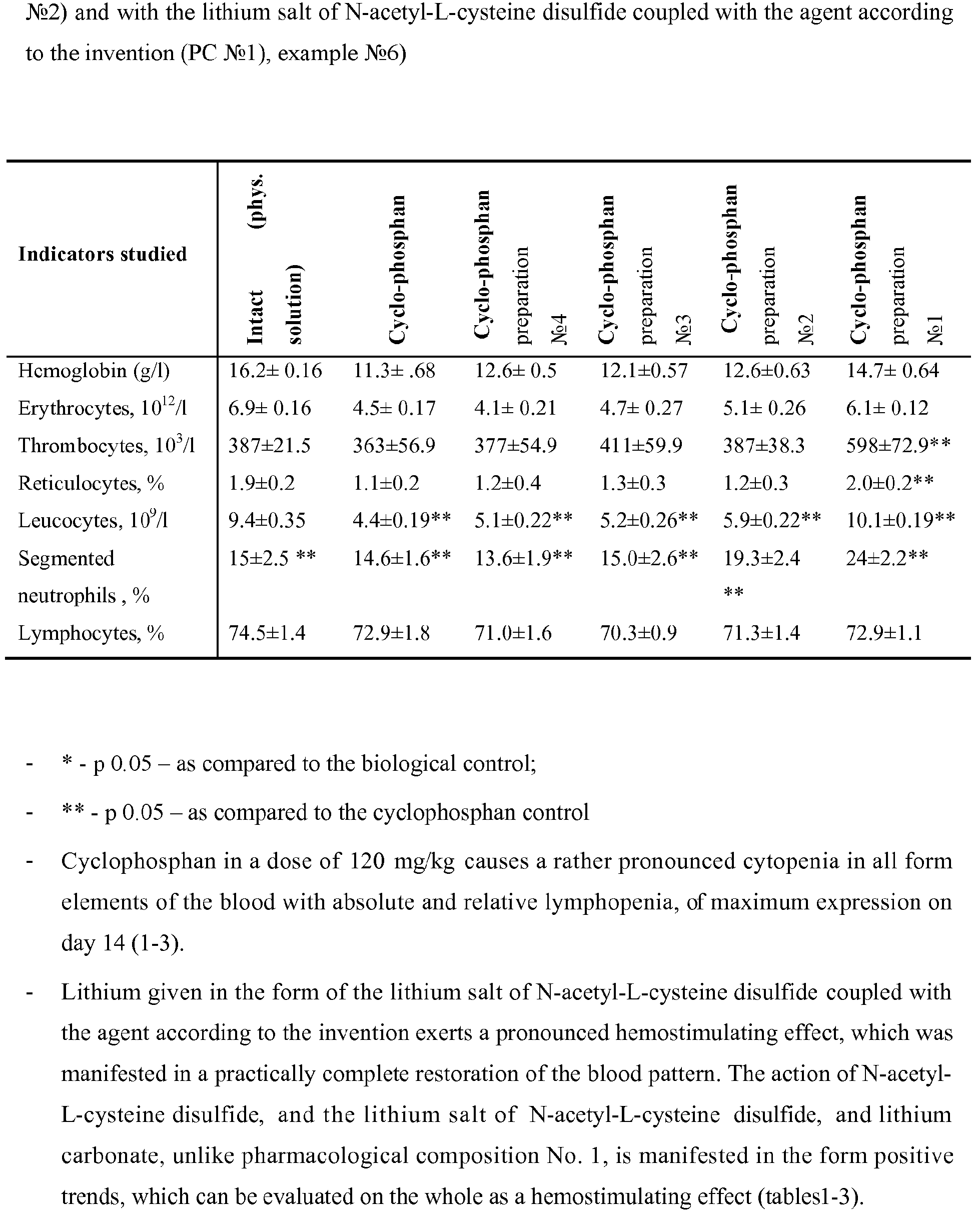

### Conclusion

The results of the studies carried out, taken altogether, let us conclude that lithium in the form of pharmacological composition Nº1, which is the lithium salt of N-acetyl-L-cysteine disulfide coupled with the agent according to the invention, has a hemostimulating action, whereas without the agent according to the invention lithium given in the form of the carbonate or N-acetyl-L-cysteine disulfide did not provide a hemostimulating action.

Thus, the agent according to the invention is characterized by an ability to potentiate the specific hemostimulating activity of lithium ions.

## Claims

1. Palladium-copper catalyst of homogeneous selective oxidation of thiols, combining a functional binuclear thiolate-bridged coordination compound of palladium (II) and a modifying thiolate complex of copper (I), having the general formula
[Pd₂^{II}(µ-SR)₂(NH₃)₄] · {Cu^{I}ₖ(SR)ₘ} (I),
where
SR is the residue of a thiolate ligand, chosen from the group including a residue of glutathione and acetylcysteine,
k = 2 to 14,
m ≥ 3k.

2. Catalyst according to claim 1, where the thiol whose oxidation is subjected to a catalytic function is N-acetyl-cysteine.

3. Catalyst according to claim 1, where the thiol whose oxidation is subjected to a catalytic function is N-glutamyl-L-cysteinyl-glycine.

4. Catalyst according to any one of claims 1-3, where the oxidation is a homogeneous selective oxidation of thiols with forming of disulfide bonds between the thiol residues.

5. Catalyst according to claim 1, obtained by the reaction of mononuclear aminate complexes of palladium (II) and corresponding thiols with complexes forming from salts of copper (II) and corresponding thiols.

6. Catalyst according to claim 1, where the molar ratio of Pd: Cu lies in the range of 1:0.1 to 1:2.

7. Catalyst according to claim 6, where the molar ratio of Pd:Cu lies in the range of 1:0.2 to 1:1.

8. Catalyst according to claim 1, for use in therapy.

9. Catalytic combination formed by a thiol chosen from among acetylcysteine, glutathione, their solvates and salts, and by the catalyst according to claim 1.

10. Combination according to claim 9, the catalyst is present in a quantity between 1·10⁻² and 1·10⁻⁷ g per mole of the thiol.

11. Combination according to claim 9, basically consisting of acetylcysteine disulfide and/or glutathione, their solvates and salts, and the catalyst according to claim 1.

12. Combination according to claim 9 for use in therapy.

13. Pharmacological combination, consisting of a catalytic combination according to claims 9-12 and a pharmacologically active compound able to enter into an addition reaction with the components of the combination according to claims 9-12.

14. Combination according to claim 13, to boost the therapeutic activity of a pharmacologically active compound

15. Combination according to claim 14, where the pharmacologically active compound is a medicinal or biologically active molecule chosen from the purine or pyrimidine bases or their derivatives.

16. Combination according to any one of claims 13-15, for use in therapy for infectious and noninfectious diseases.

17. Combination according to any one of claims 13-15, where the pharmacologically active compound is inosine.

18. Combination according to claim 15, where the pharmacologically active compound is ribavirin.

19. Pharmaceutical composition, including the catalyst according to any one of claims 1-8 or the combination according to any one of claims 9-18 and a pharmaceutically acceptable excipient.

20. Pharmaceutical composition according to claim 19, boosting the therapeutic activity of the pharmacologically active compounds.

21. Method of therapeutic action on a patient's organism to boost the therapeutic activity of a pharmacologically active compound, wherein the patient requiring this is given an effective quantity of the catalyst according to any one of claims 1-8, the combination according to any one of claims 9-12 or 13-18, or the composition according to any one of claims 19-20.
